(19) Europäisches Patentamt
European Patent Office
Office européen des brevets

(11) Publication number: **0 085 447**
**B1**

(12) # EUROPEAN PATENT SPECIFICATION

(45) Date of publication of patent specification: **21.01.87**

(51) Int. Cl.[4]: **G 03 G 5/06**, G 03 G 5/14, C 07 C 109/12

(21) Application number: **83200049.1**

(22) Date of filing: **13.01.83**

(54) **Multi-layered electrophotographic element and method of making a photocopy using such element.**

(30) Priority: **29.01.82 NL 8200331**

(43) Date of publication of application:
**10.08.83 Bulletin 83/32**

(45) Publication of the grant of the patent:
**21.01.87 Bulletin 87/04**

(84) Designated Contracting States:
**CH DE FR GB IT LI NL SE**

(56) References cited:
**DE-A-3 147 118**
**FR-A-2 335 346**
**US-A-3 290 147**

**PATENTS ABSTRACTS OF JAPAN, vol. 3, no. 106, 7th September 1979, page 96E135**

(73) Proprietor: **Océ-Nederland B.V.**
**St. Urbanusweg 43**
**NL-5914 CC Venlo (NL)**

(72) Inventor: **Everhardus, Roelof Hendrik**
**Diepstraat 9**
**Lomm (NL)**
Inventor: **Van Lomm, Gerard Jozef Everhard H.**
**Pastoor Freybeuterstraat 1A**
**Tegelen (NL)**

**EP 0 085 447 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a multi-layered electrophotographic element comprising an electrically conductive support, a photoconductive layer containing one or more radiation-sensitive charge-generating compounds, and a charge transporting layer applied to the photoconductive layer, as claimed in claim 1.

In electrophotography, an image is formed on an electrophotographic element comprising a photoconductive layer, by first providing its surface layer with a uniform electrostatic charge and then exposing it image-wise. The image-wise exposure causes the areas struck by light to become conductive and discharged. The charge remaining in the non-exposed areas forms an electrostatic latent image. This latent image is rendered visible by depositing, for example, finely divided electroscopic toner particles onto the surface layer, these particles being attracted by the remaining charge. In direct electrophotography, the image which has been rendered visible is locally fixed, e.g. by heat and/or pressure. In indirect electrophotography, the image formed on the photoconductive element is first transferred to a support, usually plain paper, and then fixed thereon. Thereupon the surface layer is cleaned from any remaining toner particles, in order to make it suitable for a subsequent copying cycle.

In practice, the electrophotographic element may consist of a charge-generating layer applied to a conductive support. Alternatively it may comprise a plurality of layers including a charge-generating layer and a charge transporting layer applied thereto.

Such multi-layered electrophotographic elements are described, for example, in U.S. patent specifications Nos. 3 713 820, 3 725 058, 3 824 099, 3 837 851, 3 839 034 and 3 898 084.

The radiation-sensitive compound in the charge-generating layer may be of an inorganic or organic nature. Where inorganic material is used, it is generally present either in the form of particles dispersed in a binder or in the form of a homogeneous film obtained, for example, by vapour deposition. Selenium is the inorganic material most used. Where organic material is used, it may, for example, be present in the form of a film-forming organic polymer, such as polyvinyl carbazole or polyvinyl pyrene for example, or in the form of finely divided pigment particles, such as bisazo-pigments, for example, of which Phenelac Blue and its derivatives belong to the best known, the said particles being dispersed in an organic binder layer.

Since pigment-binder layers have been found to have a number of disadvantages, processes have been proposed by means of which charge-generating layers can be prepared in which the radiation-sensitive compound is present in molecularly divided form instead of in the form of pigment particles. The advantage of such layers is that they can be of a much thinner and smoother nature than pigment-binder layers so that the charge transport in, and the resolving power of, such layers is also better than that of pigment-binder layers. Furthermore, the grinding operation otherwise required for their preparation can be dispensed with.

Charge-generating layers of the kind referred to here are described for example, in U.S. patent specifications Nos. 4 123 270 and 4 286 040 and in U.K. patent specification No. 1 172 355.

If it is desired to use such layers in the thinnest possible form, e.g. in a thickness of not more than 1 to 2 µm, because of the associated advantages, they must be provided with a top layer because of their great vulnerability and in order to be able to obtain the required level during uniform charging. This top layer must also permit the transport of one of the two charge carriers, usually holes, formed in the charge-generating layer during the image-wise exposure. Such top layers are required to satisfy very high requirements in this case.

Numerous kinds of charge transporting layers have already been proposed. Generally two main groups of charge transporting layers can be distinguished.

One group is formed by polymeric film-forming compound which themselves have charge transporting properties, such as for example, polyvinyl carbazole or polyvinyl pyrene. The other group is formed by charge transporting compounds dissolved in a binder which is of itself insulating.

Examples of these can be found in the above-mentioned U.S. and U.K. patent specifications.

In many cases, transporting layers of this kind also contain a so-called activator which improves the charge transporting properties of the transporting layer. Known activators are, for example, the electron acceptors trinitrofluorenone and dibenzothiophene dioxide.

A charge transporting layer should have the following properties:

| | |
|---|---|
| — mechanically strong | — adapted to be charged to a sufficient level |
| — smooth surface and good filmforming properties | — capable of transporting holes (or electrons) satisfactorily |
| — transparent to visible light | |
| — good adhesion to the generation layer | — have no or practically no injection barrier with the generation layer |
| — good retention of the electrostatic surface charge in the dark | — good cleaning properties<br>— low residual charge after exposure |

The charge transporting layers hitherto proposed fall short with respect to one or more of the above properties, so that they are less satisfactory, particularly on very thin charge-generating layers.

The object of this invention is to provide a charge transporting layer for a multi-layered electrophotographic element, which to a very high degree satisfies all the above required properties and which in respect of its low residual charge, cleaning properties and practical absense of any injection barrier, is far superior to any of the hitherto known charge transporting layers, so that it is particularly suitable especially for use on very thin charge-generating layers.

The multi-layered electrophotographic element according to the invention comprising one or more radiation-sensitive charge-generating compounds, and a charge transporting agent which is homogeneously distributed in an insulating binder and which has the general structural formula:

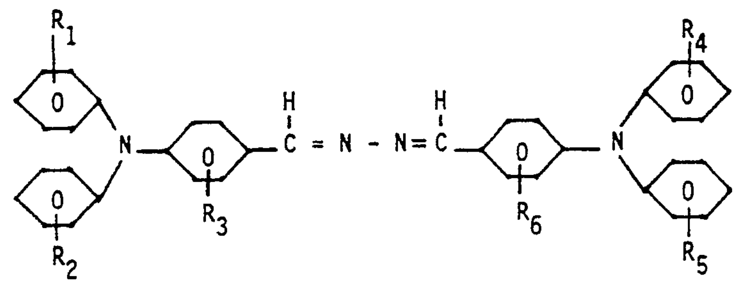

Wherein $R_1$ to $R_6$ inclusive, which may be the same or different, represent a hydrogen atom or an alkyl group having 1 to 4 carbon atoms.

Preference, however, is given to compounds of the general structural formula wherein $R_3$ and $R_6$ represent a hydrogen atom or an alkyl group having 1 to 4 carbon atoms and $R_1$, $R_2$, $R_4$ and $R_5$ represent an alkyl group having 1 to 4 carbon atoms in the para position.

The charge transporting layer of the element of the invention may also comprise a mixture of two or more compounds according to the general structural formula.

The symmetrical and asymmetrical azines according to the general structural formula can be synthesized by generally known methods for related compounds, such as e.g. described in Houben-Weyl, Methoden der organischen Chemie, 4th edition, Vol. 10/2, pages 89 to 111, published by George Thieme Verlag, Stuttgart in 1967.

Particularly good results were obtained with the compound belonging to this group according to structural formula 2 of the formula sheet.

Electrophotographic elements according to the invention the charge transporting layer of which comprises said compound, viz. 4-[bis(4-methylphenyl)amino] benzaldehyde, azine as charge generating agent, show a high photo-sensitivity, a very low residual charge after exposure and a low dark decay, even when they are charged to not more than 30—70% of their maximum apparent surface voltage. (ASVmax). This aspect is of great advantage since electrophotographic elements which are charged to a level well below their $ASV_{max}$ have a substantially higher permanence than elements which are charged to their $ASV_{max}$.

Furthermore, cleaning of the charge transporting layer after transfer of the developed image was found to give rise to no problems, as compared with multi-layered elements of the kind in question here, in which the charge transporting contained one of the hitherto known charge transporting agents.

The quantity of charge transporting agent in the charge transporting layer of the electrophotographic element according to the invention may vary within wide limits. It generally lies between 15% by weight and 70% by weight, based on the total quantity of solids and preferably between 20% by weight and 40% by weight.

The insulating binder used for the charge transporting layer of the element according to the invention may be any material suitable for that purpose, e.g. polystyrenes, silicone resins, polyesters of acrylic and methacrylic acid, vinyl polymers and vinyl copolymers. Particularly good results are obtained with polycarbonates, because of their high transparency, mechanical strength and good adhesion to the photosensitive layer.

Any support known for that purpose can be used in the electrophotographic element according to the invention.

Such supports may be conductive by themselves, e.g. supports of aluminium, steel or nickel, or they may be rendered conductive, such as e.g. paper or plastic supports to which a thin conductive layer, e.g. of aluminium or nickel, has been applied. For use in indirect electrophotography, for which the present element is eminently suitable because of its particular properties, the support will generally have an endless form, e.g. a drum or a flexible web of paper or plastic, the ends of which are interconnected.

The radiation-sensitive charge-generating compound to be used in the photoconductive layer of the element according to the invention may be of either an inorganic or organic nature. Examples of the former are selenium or amorphous silicon. Preferably, however, organic compounds and more particularly radiation-sensitive bisazo compounds are applied. Examples of radiation-sensitive layers containing one or

more bisazo compounds in molecularly divided form will be found in the above-mentioned U.S. patent specification No. 4 123 270 and Applicant's own U.S. patent specification No. 4 286 040.

The thickness of the photoconductive layer is preferably between about 0,2 and 2 µm.

In a particular embodiment, the charge transporting layer of the electrophotographic element according to the invention comprises one or more activators. Particularly if it is desired to charge the element only partially, to say 30—70% of its $AVS_{max}$, in order to obtain a higher permanence, the use of an activator is usually desirable to improve the discharge characteristic. In principle it is possible to use any of the activators known for that purpose.

Examples of usable activators are trinitro-fluorenone, the dibenzothiophene oxides mentioned in U.S. patent specification 3 905 814 and the N-(fluoren-9-ylidene) anilines mentioned in U.S. patent specification No. 3 935 009.

Particularly good results were obtained with the activators terephthalal dimalonitrile (TDM: formula 3 of the formula sheet) and 1,3,7-trinitro-dibenzothiophene-5,5-dioxide (DBTO: formula 11 of the formula sheet).

Unlike many of the activators used hitherto, TDM is in addition absolutely non-mutagenic.

The quantity of activator required is generally between 1 and 15% by weight based on the charge transporting agent. If TDM is used in combination with a charge transporting agent according to the invention it has been found that quantities of between 0,5 and 3% by weight are sufficient for the required results.

The electrophotographic element according to the invention can be prepared by one of the methods described in the patent specifications referred to hereinbefore. Both the preparation of the charge-generating layer and of the charge transporting layer are described in detail therein.

The azines according to the general structural formula 1 which are used in the charge transporting layer according to the invention can be synthesized by condensation of the corresponding *p*-(diarylamino)-benzaldehydes (which may or may not contain alkyl groups) with hydrazine. The preparation method is similar to that of the condensation of *p*-(dimethylamino)-benzaldehyde and hydrazine described in Beilstein (Basic Series *14*, 36.

Most of the aldehydes referred to can be prepared by formylation of the corresponding triarylamines by means of N,N-dimethylformamide and phosphoroxytrichloride. These reactions can be carried out similarly to the preparation of *p*-(dimethylamino) benzaldehyde (Org. Synth. Coll. Vol. IV, p. 331). The preparation of *p*-(diphenylamino)benzaldehyde is described in J. Org. Chem. *30*, 3714 (1965).

Electrophotographic elements containing a charge transporting layer according to the invention applied to a thin charge-generating layer are eminently suitable for use as a so-called permanent master in an indirect electro-photographic copying process. It is precisely here that their particular advantages referred to hereinbefore are fully manifest and copies of high quality can be obtained even with partial charging.

The latent image formed on the charge transporting layer can be rendered visible either by means of a two-component or a one-component developer. In the former case the developer consists of coarser carrier particles, usually iron, and very finely divided toner particles which obtain the required polarity upon contact with the carrier particles. In the second case the developer consists essentially of finely divided toner particles which may be conductive ($\rho < 10^{10}$ Ohm.m.) or insulating ($\rho > 10^{10}$ Ohm.m).

The electrophotographic element according to the invention appears to be particularly suitable for development by means of a one-component developer, and this has a number of advantages. When an insulating one-component developer is used, it has however been found desirable to provide the electrophotographic element according to the invention with a function layer in the form of a raster.

This kind of layers and the location and method of applying them are known to those skilled in the art. They are described, inter alia, in "Xerography and related processes" by Dessauer and Clark, 1965, pp. 11—117.

The invention will be explained in detail with reference to the following examples.

Example 1

Preparation of the charge-generating layer

In order to test the charge-transporting layers according to the invention, a charge-generating layer was prepared which contained as charge-generating compound the bisazo dye 4,4'[(3,3'-dimethoxy[1,1'-biphenyl]-4,4'-diyl) bis (azo)] bis [3-hydroxy-N-phenyl-2-naphthalene carboxamide] (formula 6 of the formula sheet) distributed in molecularly divided form in a binder.

For that purpose, the following solutions were first prepared:

60 ml of 2% polymeric cellulose acetate butyrate in acetone,

13 ml of N,N-dimethylformamide + 1 g of naphthol according to formula 4 of the formula sheet.

7 ml of N,N-dimethylformamide + 0.5 g of a diazonium compound according to formula 5 of the formula sheet.

After 10 minutes storage in the dark, the solution was applied to a conductive support (Melinex with a vapour-deposited aluminium layer) by dip-coating at 25 to 30°C and 30—40% relative humidity. After drying, the in-situ coupling to the above-mentioned bisazo compound was effected in an ammonia development unit.

The thickness of the charge-generating layer was 0.5 µm.

Example 2

A charge transporting layer comprising the azine according to formula 2 of the formula sheet as the charge transporting compound was applied by dip-coating to a charge-generating layer prepared according to Example 1.

Application was effected by means of a solution of 25 ml of 10% "Lexan 141" (a polycarbonate binder from General Electric) in 1,2-dichloroethane, 1.5 g of the said azine and 8 ml of tetrahydrofuran. After 15 minutes drying to the air the resulting double layer was dried in vacuo at 135°C for 30 minutes. By means of the resulting multi-layered electrophotographic element photocopies were made in an indirect photocopying machine.

The following points were checked: layer thickness, adhesion of the layers, charging, dark-decay, lightsensitivity, surface charge density, residual potential, memory effect, image quality of the copy obtained after transfer to plain paper of the image rendered visible on the charge transporting layer by means of a one-component developer, followed by fixation by means of heat and pressure, and permanence.

The results obtained with the electrophotographic element according to this example, and the results obtained with elements according to the examples 3 to 15, are summarized after Example 15.

Example 3

Same as Example 2 except that 0.02 g of the activator TDM (formula 3 of the formula sheet) was dissolved in the said 8 ml of tetrahydrofuran.

Example 4

Same as Example 2 except that 0.12 g of the activator TDM (formula 3 of the formula sheet) was dissolved in the said 8 ml of tetrahydrofuran.

Example 5

Same as Example 2 except that 0.02 g of the activator DBTO (formula 11 of the formula sheet) was dissolved in the said 8 ml of tetrahydrofuran.

Example 6

Same as Example 2 except that 0.12 g of the activator DBTO (formula 11 of the formula sheet) was dissolved in the said 8 ml of tetrahydrofuran.

Example 7

Same as Example 2 except that the said azine according to formula 2 of the formula sheet was replaced by the azine according to formula 7 of the formula sheet and 0.02 g of the activator TDM (formula 3 of the formula sheet) was dissolved in the said 8 ml of tetrahydrofuran.

Example 8

Same as Example 2 except that the said azine according to formula 2 of the formula sheet was replaced by the azine according to formula 8 of the formula sheet and 0.02 g of the activator TDM (formula 3 of the formula sheet) was dissolved in the said 8 ml of tetrahydrofuran. For better solubility of the azine 7 ml of 1,2-dichloroethane were also added.

Example 9

Same as Example 2 except that the azine referred to therein was replaced by 0.75 g of the azine according to formula 9 of the formula sheet and 0.01 g of the activator TDM (formula 3 of the formula sheet) was dissolved in the said 8 ml of tetrahydrofuran.

Example 10 (Comparative example)

Same as Example 2 except that the said azine according to formula 2 of the formula sheet was replaced by the oxadiazole according to formula 10 of the formula sheet and no tetrahydrofuran was added.

Example 11 (Comparative example)

Same as Example 2 except that the said azine according to formula 2 of the formula sheet was replaced by the oxadiazole according to formula 10 of the formula sheet and 0.02 g of the activator TDM (formula 3 of the formula sheet) was dissolved in the said 8 ml of tetrahydrofuran.

Example 12 (Comparative example)

Same as Example 2 except that the said azine according to the formula 2 of the formula sheet was replaced by the oxadiazole according to formula 10 of the formula sheet and 0.12 g of TDM (formula 3 of the formula sheet) was dissolved in the said 8 ml of tetrahydrofuran.

Example 13 (Comparative example)

Same as Example 2 except that the said azine according to formula 2 of the formula sheet was replaced by the triarylmethane according to formula 12 of the formula sheet.

Example 14 (Comparative example)

Same as Example 2 except that the said azine according to formula 2 of the formula sheet was replaced by the triarylmethane according to formula 12 of the formula sheet and 0.02 g of the activator TDM (formula 3 of the formula sheet) was dissolved in the said 8 ml of tetrahydrofuran.

Example 15 (Comparative example)

Same as Example 2 except that the said azine according to formula 2 of the formula sheet was replaced by the triarylmethane according to formula 12 of the formula sheet and 0.12 g of the activator TDM (formula 3 of the formula sheet) was dissolved in the said 8 ml of tetrahydrofuran.

Results of Examples 2 to 15

The charge transporting layers of the Examples were all 3 to 4 µm thick. The adhesion of the layers was excellent. The memory effect of the fully charged layers of Examples 2 to 15 was very low. Upon partial charging, the memory effect of the layers of Examples 2 to 15 was completely absent. The copy quality for both full and partial charging of the layers of Examples 7, 9 to 15 was good, and of the layers of Examples 2, 3, 4, 5, 6 and 8 was excellent. For all layers the permanence upon partial charging was considerably higher than upon full charging. The layers of the examples 2 to 9 could all easily be cleaned when a one-component developer was used for the development of the latent electrostatic image, whilst the layers of the comparative examples 10 to 15 could only be cleaned with great difficulties.

The photo-electric results upon full charging are summarized in Table 1. The photo-electric results upon partial charging are summarized in Table 2.

TABLE 1

| Example | Charge transporting layer | | Maximum charging | | | | |
|---|---|---|---|---|---|---|---|
| | Charge transporting agent | Activator | ASV volt | DD–1 % | L–25 $mJ/m^2$ | sigma $mC/m^2$ | residual % |
| 2 | 1.5 g formula 2 | – | –437 | 13 | 44 | 4.1 | 5 |
| 3 | 1.5 g formula 2 | 0.02 g TDM | –501 | 12 | 33 | 4.3 | 5 |
| 4 | 1.5 g formula 2 | 0.12 g TDM | –417 | 13 | 30 | 4.2 | 4 |
| 5 | 1.5 g formula 2 | 0.02 g DBTO | –519 | 10 | 38 | 4.5 | 4 |
| 6 | 1.5 g formula 2 | 0.12 g DBTO | –445 | 12 | 36 | 4.3 | 3 |
| 7 | 1.5 g formula 7 | 0.02 g TDM | –547 | 8 | 54 | 4.7 | 10 |
| 8 | 1.5 g formula 8 | 0.02 g TDM | –341 | 11 | 33 | 4.5 | 7 |
| 9 | 0.75g formula 9 | 0.01 g TDM | –450 | 9 | 60 | 4.5 | 13 |
| 10 | 1.5 g formula 10 | – | –422 | 12 | 56 | 4.2 | 11 |
| 11 | 1.5 g formula 10 | 0.02 g TDM | –369 | 13 | 36 | 4.1 | 8 |
| 12 | 1.5 g formula 10 | 0.12 g TDM | –344 | 13 | 52 | 4.2 | 7 |
| 13 | 1.5 g formula 12 | – | –420 | 12 | 56 | 4.2 | 7 |
| 14 | 1.5 g formula 12 | 0.02 g TDM | –412 | 13 | 43 | 4.1 | 6 |
| 15 | 1.5 g formula 12 | 0.12 g TDM | –355 | 10 | 47 | 4.1 | 10 |

ASV: Apparent surface voltage in volts after charging.
DD—1: Dark decay in the first second as a percentage of the ASV.
L—25: Quantity of light in $mJ/m^2$ in order to discharge the layer with a BRAUN flash type F900 to 25% of the ASV.
sigma: Surface charge density in $mC/m^2$, measured after 1 second dark decay.
Residual: Percentage of the ASV remaining after exposure with 100 $mJ/m^2$ (BRAUN flash type F900).

TABLE 2

| Example | Charge transporting layer | | Partial charging (about 280 Volts) | | | | |
|---|---|---|---|---|---|---|---|
| | Charge transporting agent | Activator | ASV volts | DD—1 % | L—25 mJ/m² | sigma mC/m² | residual % |
| 2 | 1.5 g formula 2 | – | –285 | 3 | 44 | 2.5 | 6 |
| 3 | 1.5 g formula 2 | 0.02 g TDM | –273 | 2 | 30 | 2.4 | 6 |
| 4 | 1.5 g formula 2 | 0.12 g TDM | –288 | 5 | 26 | 2.9 | 6 |
| 5 | 1.5 g formula 2 | 0.02 g DBTO | –289 | 3 | 32 | 2.5 | 5 |
| 6 | 1.5 g formula 2 | 0.12 g DBTO | –282 | 2 | 33 | 2.6 | 5 |
| 7 | 1.5 g formula 7 | 0.02 g TDM | –287 | 5 | 40 | 2.3 | 16 |
| 8 | 1.5 g formula 8 | 0.02 g TDM | –280 | 7 | 30 | 3.5 | 6 |
| 9 | 0.75g formula 9 | 0.01 g TDM | –282 | 5 | 50 | 3.1 | 8 |
| 10 | 1.5 g formula 10 | – | –281 | 2 | 70 | 2.7 | 16 |
| 11 | 1.5 g formula 10 | 0.02 g TDM | –285 | 3 | 33 | 3.1 | 9 |
| 12 | 1.5 g formula 10 | 0.12 g TDM | –274 | 5 | 44 | 3.4 | 8 |
| 13 | 1.5 g formula 12 | – | –279 | 2 | 68 | 2.7 | 11 |
| 14 | 1.5 g formula 12 | 0.02 g TDM | –283 | 3 | 33 | 3.1 | 9 |
| 15 | 1.5 g formula 12 | 0.12 g TDM | –273 | 6 | 36 | 3.3 | 11 |

ASV: Apparent surface voltage in volts after charging.
DD—1: Dark decay in the first second as a percentage of the ASV.
L—25: Quantity of light in mJ/m² in order to discharge the layer with a BRAUN flash type F900 to 25% of the ASV.
sigma: Surface charge density in mC/m², measured after 1 second dark decay.
Residual: Percentage of the ASV remaining after exposure with 100 mJ/m² (BRAUN flash type F900).

Example 16

A charge-generating layer was prepared which comprised as charge-generating compound the bis-azo dye according to formula 6 of the formula sheet in the form of small pigment particles (approx. 0,2 µm) homogenously distributed in a binder.

For that purpose 1 g of the said bis-azo dye was dispersed in 50 ml of 2% polymeric cellulose acetate butyrate in acetone by milling during 24 hours in a ball mill. The dispersion so obtained was applied by dip-coating to a conductive support (Melinex with a vapour-disposited aluminium layer).

The thickness of this charge-generating layer after drying was 1,5 μm.

To this charge-generating layer a charge transporting layer was applied by dip-coating, by means of the azine according to formula 2 of the formula sheet as the charge transporting agent. The application was effected by means of a solution of 25 ml of 10% "Lexan 141" in 1,2-dichloro-ethane, 1.5 g of said azine and 8 ml of tetrahydrofuran in which 0.03 g of the activator TDM (formula 3 of the formula sheet) had been dissolved. After 15 minutes drying to the air the resulting double layer was dried in vacuo at 135°C for 30 minutes.

By means of the resulting multi-layered electrophotographic element photocopies were made in an indirect photocopying machine.

The following points were checked: layer thickness, adhesion of the layers, charging, dark-decay, lightsensitivity, surface charge density, residual potential, memory effect, image quality of the copy obtained after transfer to plain paper of the image rendered visible on the charge transporting layer by means of a one-component developer, followed by fixation by means of heat and pressure, cleanability of the charge transporting layer, and permanence.

The results obtained with the electrophotographic element according to this example, as well as the results obtained with the elements according to the examples 17 to 21, are summerized after example 21.

Example 17 (Comparative example)

Same as example 16 except that instead of the azine according to formula 2 of the formula sheet, the tri-arylmethane according to formula 12 of the formula sheet was now applied in the charge transporting layer.

Example 18

A charge generating layer was prepared comprising as charge-generating compounds the bis-azo dye according to formula 13 and the polymeric dye according to formula 14 of the formula sheet, dispersed as small pigment particles (approx. 0.2 μm) in a binder which also comprised a charge transporting compound.

For that purpose 2 g of "Lexan 141" and 1.2 g of the azine according to formula 2 of the formula sheet were successively dissolved in a mixture of 20 ml of tetrahydrofuran and 40 ml of 1,2-dichloroethane.

Subsequently 1 g of the said bis-azo dye together with 1 g of the said polymeric dye were homogeneously distributed in the solution by milling in a ball mill during 24 hours.

The dispersion so obtained was applied by dip-coating to a conductive support (Melinex with a vapour-deposited aluminium layer).

After drying the thickness of the charge-generating layer was 1.3 μm. Analogous to Example 16, a charge transporting layer was applied to the charge-generating layer, by dip-coating by means of the azine according to formula 2 of the formula sheet.

Example 19 (Comparative example)

Same as example 18, except that the tri-aryl methane according to formula 12 of the formula sheet instead of the said azines according to formula 2 of the formula sheet was used in the charge-generating layer as well as in the charge transporting layer.

Example 20

A charge-generating layer was prepared comprising the metal-free phthalocyanine according to formula 15 of the formula sheet as the charge-generating compound.

For this purpose a thin layer of the metal-free phthalocyanine was deposited on a conductive support (Melinex with a vapour-deposited aluminium layer) by evaporation at a pressure of $10^{-5}$ to $10^{-6}$ torr. Analogous to Example 16, a charge transporting layer was applied to the charge-generating layer by dip-coating by means of the azine according to formula 2 of the formula sheet.

Example 21 (Comparative example)

Same as example 20 except that the tri-arylmethane according to formula 12 of the formula sheet instead of the said azine according to formula 2 of the formula sheet was used in the charge transporting layer.

Results of the Examples 16 to 21

The charge transporting layers from the examples all had a thickness of approximately 4 μm. The adhesion of the layers was excellent. The memory effect of the fully charged layers of the Examples 16 to 21 was extremely low. At partial charging the memory effect was completely absent.

The layers of the examples 16, 18 and 20 could easily be cleaned after development with a one-component developer. The layers of the comparative examples 17, 19 and 21 to the contrary could only be cleaned with great difficulties.

The copy quality for both full and partial charging of the layers of the Examples 17, 19 and 21 was good and of the layers of the Examples 16, 18 and 20 excellent. For all layers the permanence upon partial charging was substantially higher than upon full charging. The photo-electric results upon full charging are summarized in table 3. The photo-electric results upon partial charging are summarized in table 4.

TABLE 3

| Example | Electrophotographic element | | Maximum charging | | | | |
|---|---|---|---|---|---|---|---|
| | Charge-generating compound | Charge-transporting agent | ASV volts | DD–1 % | L–25 mJ/m² | sigma mC/m² | residual % |
| 16 | Pigment: formula 6 | formula 2 | –604 | 8 | 41 | 4.1 | 10 |
| 17 | Pigment: formula 6 | formula 12 | –542 | 8 | 79 | 4.6 | 16 |
| 18 | Pigment: formula 13 + formula 14 | formula 2 | –631 | 13 | 41 | 3.3 | 9 |
| 19 | Pigment: formula 13 + formula 14 | formula 12 | –711 | 12 | 49 | 3.4 | 11 |
| 20 | deposited by evaporation: formula 15 | formula 2 | –445 | 19 | 35 | 5.1 | 1 |
| 21 | deposited by evaporation: formula 15 | formula 12 | –550 | 11 | 38 | 5.3 | 3 |

TABLE 4

| Example | Electrophotographic element | | Partial charging (about 300 volts) | | | | |
|---|---|---|---|---|---|---|---|
| | Charge-generating compound | Charge-transporting agent | ASV volts | DD–1 % | L–25 mJ/m² | sigma mC/m² | residual % |
| 16 | Pigment: formula 6 | formula 2 | –291 | 3 | 31 | 2.0 | 7 |
| 17 | Pigment: formula 6 | formula 12 | –288 | 2 | 80 | 2.1 | 18 |
| 18 | Pigment: formula 13 + formula 14 | formula 2 | –302 | 5 | 40 | 1.6 | 14 |
| 19 | Pigment: formula 13 + formula 14 | formula 12 | –313 | 4 | 48 | 1.5 | 18 |
| 20 | deposited by evaporation: formula 15 | formula 2 | –299 | 7 | 30 | 3.7 | 2 |
| 21 | deposited by evaporation: formula 15 | formula 12 | –295 | 2 | 44 | 3.1 | 8 |

ASV: Apparent surface voltage in volts after charging.
DD—1: Dark decay in the first second as a percentage of the ASV.
L—25: Quantity of light in $mJ/m^2$ in order to discharge the layer with a BRAUN flash type F900 to 25% of the ASV.
sigma: Surface charge density in $mC/m^2$, measured after 1 second dark decay.
Residual: Percentage of the ASV remaining after exposure with 100 $mJ/m^2$ (BRAUN flash type F900).

Formula sheet

1. 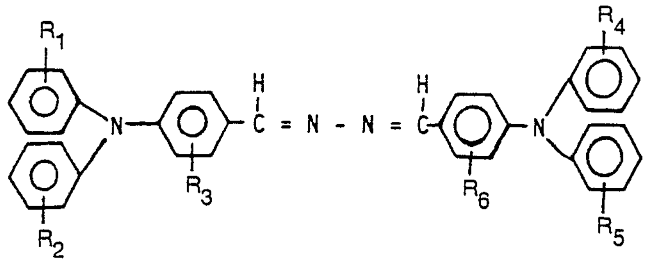

2. 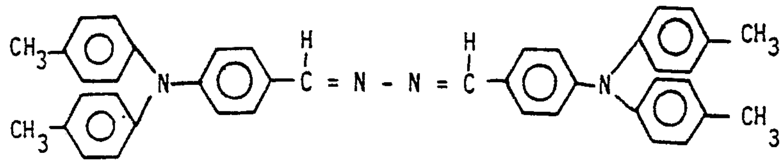

3. 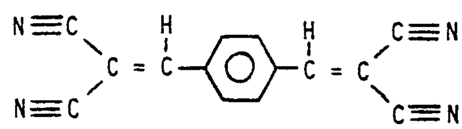

4. 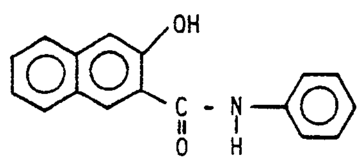

5. 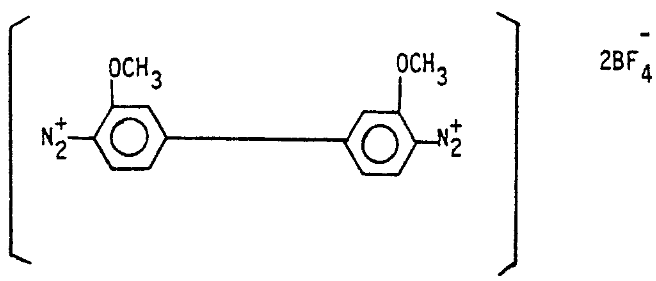

6. 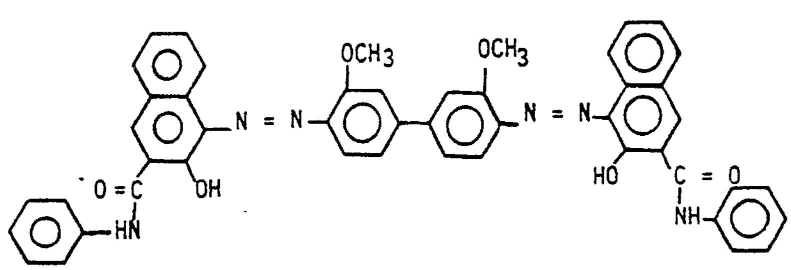

7. 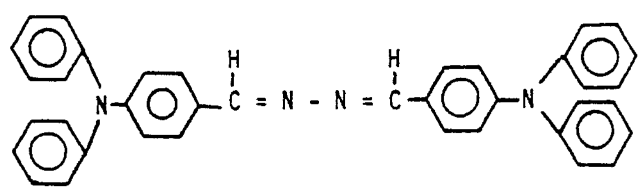

Formula sheet (continued)

8. 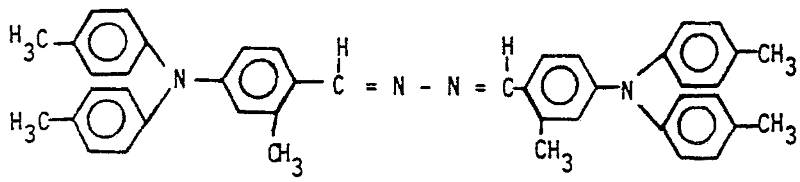

9. 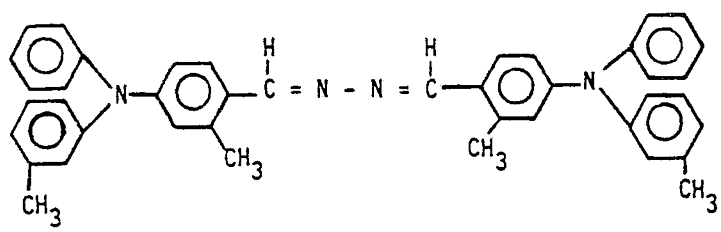

10. 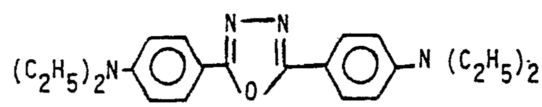

11. 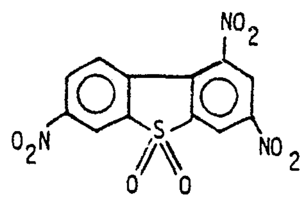

12. 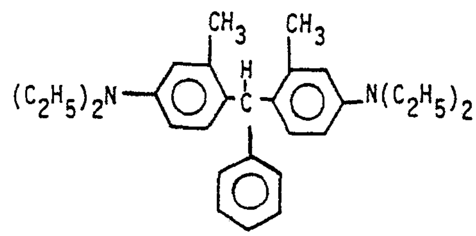

13. 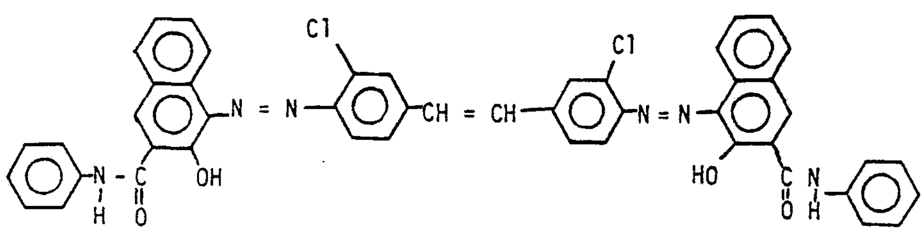

14. 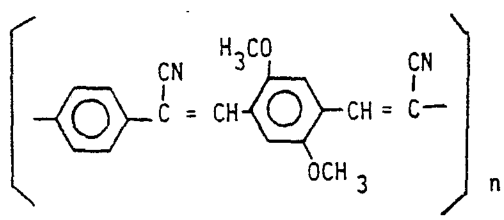

Formula sheet (continued)

15. 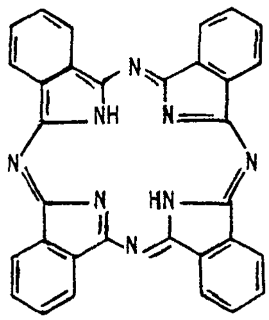

## Claims

1. Multi-layered electrophotographic element comprising an electrically conductive support, a photoconductive layer comprising one or more radiation-sensitive charge-generating compounds, and a charge transporting layer applied to the photoconductive layer, characterised in that the charge transporting layer comprises an azine as charge transporting agent which is homogeneously distributed in an insulating binder and which has the general structural formula:

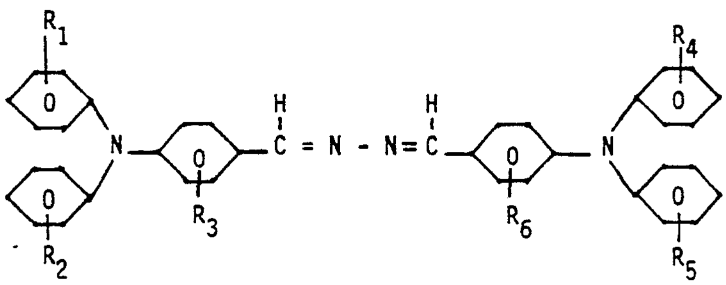

wherein $R_1$ to $R_6$ inclusive, which may be the same or different, represent a hydrogen atom or alkyl group having 1 to 4 carbon atoms.

2. An element according to claim 1, characterised in that the charge transporting layer comprises a charge transporting agent according to the general structural formula wherein $R_3$ and $R_6$ represent a hydrogen atom or an alkyl group having 1 to 4 carbon atoms and $R_1$, $R_2$, $R_4$ and $R_5$ represent an alkyl group having 1 to 4 carbon atoms in the para position.

3. An element according to claim 2, characterised in that the charge transporting layer comprises a charge transporting agent according to the general structural formula in which $R_1$, $R_2$, $R_4$ and $R_5$ represent a methyl group.

4. An element according to claim 3, characterised in that the charge transporting agent has the structural formula:

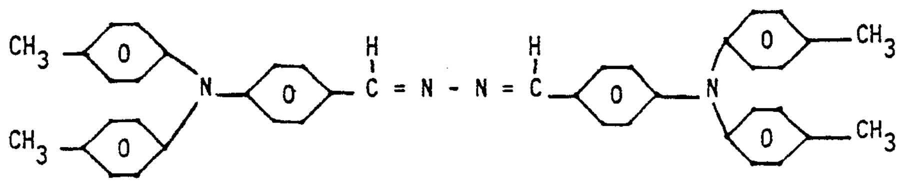

5. An element according to claim 1, 2, 3 or 4, characterised in that the charge transporting layer also comprises an activator acting on the charge transporting agent.

6. An element according to claim 5, characterised in that the activator has the structural formula:

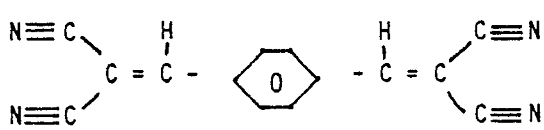

7. An element according to any of the preceding claims, characterised in that the charge transporting layer is applied to a charge-generating layer which is applied to an endless support and which comprises one or more radiation-sensitive charge-generating compounds in molecularly divided form.

8. A method of making a photocopy, in which an element according to claim 5, 6 or 7 is uniformly charged and then exposed image-wise, whereafter the resulting latent image is developed and is transferred on to a support, on which it is fixed, characterised in that the element is charged to at most 70% of its maximum chargeability.

**Patentansprüche**

1. Mehrschichtiges elektrophotographisches Element mit einem elektrisch leitenden Träger, einer photoleitenden Schicht, die eine oder mehrere strahlungsempfindliche, ladungserzeugende Verbindungen enthält, und einer auf der photoleitenden Schicht aufgebrachten Ladungstransportschicht, dadurch gekennzeichnet, dass die Ladungstransportschicht ein Azin als Ladungen transportierendes Mittel enthält, welches in einem isolierenden Bindemittel homogen verteilt ist und der allgemeinen Formel

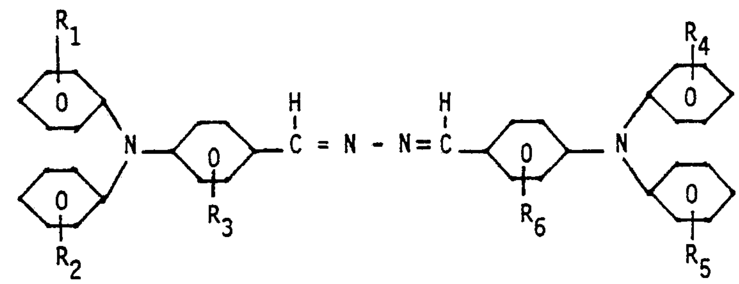

entspricht, in der $R_1$ bis $R_6$ einschliesslich, die gleichartig oder verschieden sein können, Wasserstoffatome oder Alkylgruppen mit 1 bis 4 Kohlenstoffatomen bedeuten.

2. Element nach Anspruch 1, dadurch gekennzeichnet, dass die Ladungstransportschicht ein Ladungen transportierendes Mittel der allgemeinen Formel enthält, in der $R_3$ und $R_6$ Wasserstoffatome oder Alkylgruppen mit 1 bis 4 Kohlenstoffatomen und $R_1$, $R_2$, $R_4$ und $R_5$ jeweils Alkylgruppen mit 1 bis 4 Kohlenstoffatomen in der para-Stellung bedeuten.

3. Element nach Anspruch 2, dadurch gekennzeichnet, dass die Ladungstransportschicht ein Ladungen transportierendes Mittel der allgemeinen Formel enthält, in der $R_1$, $R_2$, $R_4$ und $R_5$ jeweils Methylgruppen bedeuten.

4. Element nach Anspruch 3, dadurch gekennzeichnet, dass das Ladungen transportierende Mittel der folgenden Formel entspricht:

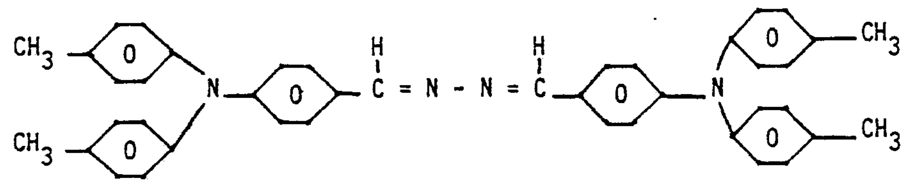

5. Element nach den Ansprüchen 1, 2, 3 oder 4, dadurch gekennzeichnet, dass die Ladungstransportschicht zusätzlich einen auf das Ladungen transportierende Mittel einwirkenden Aktivator enthält.

6. Element nach Anspruch 5, dadurch gekennzeichnet, dass der Aktivator der folgenden Formel entspricht:

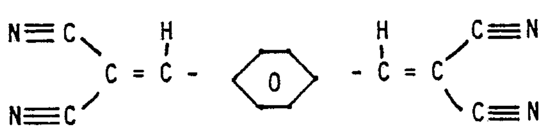

7. Element nach einem der vorhergehenden Ansprüche, dadurch gekennzeichnet, dass die Ladungstransportschicht auf eine ladungserzeugende Schicht aufgebracht ist, die auf einem endlosen Träger vorliegt und eine oder mehrere strahlungsempfindliche, ladungserzeugende Verbindungen in molekular verteilter Form umfasst.

8. Verfahren zur Erzeugung einer Photokopie, gemäss dem ein Element nach Anspruch 5, 6 oder 7 gleichförmig aufgeladen und dann bildmässig belichtet wird, wonach das resultierende latente Bild entwickelt und auf einen Träger übertragen wird, auf welchem es fixiert wird, dadurch gekennzeichnet, dass das Element auf höchstens 70% seiner maximalen Aufladbarkeit aufgeladen wird.

**Revendications**

1. Elément électrophotographique multicouche comprenant un support électriquement conducteur, une couche photoconductrice comprenant un ou plusieurs composés générateur de charges, sensibles au

rayonnement, et une couche de transport de charges appliquée sur la couche photoconductrice, caractérisé en ce que la couche de transport de charges comprend une azine comme agent de transport de charges qui est répartie de manière homogène dans un liant isolant, et qui répond à la formule développée générale:

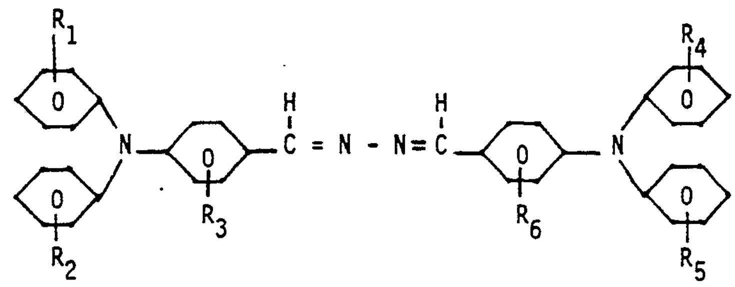

dans laquelle $R_1$ à $R_6$ compris, qui peuvent être identiques ou différents, représentent chacun un atome d'hydrogène ou un groupe alkyle ayant de 1 à 4 atomes de carbone.

2. Elément suivant la revendication 1, caractérisé en ce que la couche de transport de charges comprend un agent de transport de charge suivant la formule développée générale dans laquelle $R_3$ et $R_6$ représentent chacun un atome d'hydrogène ou un groupe alkyle ayant 1 à 4 atomes de carbone et $R_1$, $R_2$, $R_4$ et $R_5$ représentent chacun un groupe alkyle ayant 1 à 4 atomes de carbone dans la position para.

3. Elément suivant la revendication 2, caractérisé en ce que la couche de transport de charges comprend un agent de transport de charges suivant la formule développée générale dans laquelle $R_1$, $R_2$, $R_4$ et $R_5$ représentent chacun un groupe méthyle.

4. Elément suivant la revendication 3, caractérisé en ce que l'agent de transport de charges répond à la formule développée:

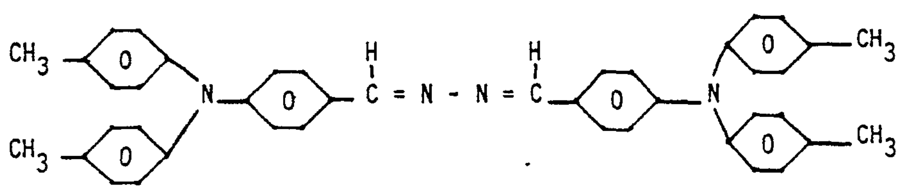

5. Elément suivant les revendications 1, 2, 3 ou 4, caractérisé en ce que la couche de transport de charges comprend aussi un activateur agissant sur l'agent de transport de charges.

6. Elément suivant la revendication 5, caractérisé en ce que l'activateur répond à la formule développée:

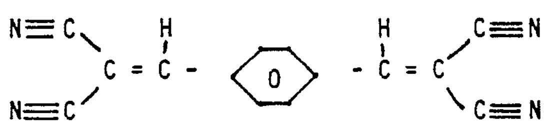

7. Elément suivant l'une quelconque des revendications précédentes, caractérisé en ce que la couche de transport de charges est appliquée sur une couche génératrice de charges qui est appliquée sur un support sans fin et qui comprend un ou plusieurs composés générateurs de charges sensibles au rayonnement, sous une forme moléculairement divisée.

8. Procédé de réalisation d'une photocopie, dans lequel un élément conforme aux revendications 5, 6 ou 7 est chargé uniformément puis exposé selon une image, après quoi l'image latente obtenue est développée puis transférée sur un support, sur lequel elle est fixée, caractérisé en ce que l'élément est chargé à 70% au plus de sa capacité de charge maxima.